# EUROPÄISCHE PATENTSCHRIFT

(11) **EP 1 144 363 B1**
(45) Veröffentlichungstag und Bekanntmachung des Hinweises auf die Patenterteilung: **20.10.2004**
(21) Anmeldenummer: 99968384.0
(22) Anmeldetag: 23.12.1999
(51) Int. Cl.: C07C 231/10

(54) **HERSTELLUNG VON FORMAMID UNTER VERWENDUNG VON NATRIUMDIFORMYLAMID**
PRODUCTION OF FORMAMIDE USING SODIUM DIFORMYLAMIDE
PREPARATION DE FORMAMIDE A L'AIDE DE DIFORMYLAMIDE DE SODIUM

(30) Priorität: 18.01.1999 DE 19901744
(43) Veröffentlichungstag der Anmeldung: 17.10.2001
(73) Patentinhaber: BASF AKTIENGESELLSCHAFT, 67056 Ludwigshafen (DE)
(72) Erfinder: DAHLHAUS, Jürgen, D-67117 Limburgerhof (DE); KARL, Jörn, D-68159 Mannheim (DE); SCHULZ, Michael, D-67067 Ludwigshafen (DE); WENZEL, Anne, D-76676 Graben-Neudorf (DE); HARDER, Wolfgang, D-69469 Weinheim (DE); HÖHN, Arthur, D-67281 Kirchheim (DE)
(74) Vertreter: Isenbruck, Günter
(86) Internationale Anmeldenummer: PCT/EP1999/010384
(87) Internationale Veröffentlichungsnummer: WO 2000/041995

(56) Entgegenhaltungen:
- DE-A- 2 710 725
- GB-A- 823 778
- US-A- 3 781 352
- I. ELVERS ET AL.: "Formamides to hexamethylenediamine" ULLMANN'S ENCYCLOPEDIA OF INDUSTRIAL CHEMISTRY, Bd. A12, 1989, XP002137322

## Beschreibung

Die Erfindung betrifft ein Verfahren zur Herstellung von Formamid durch Umsetzung von Ammoniak und Kohlenmonoxid.

Formamid (NH₂CHO) ist ein ausgezeichnetes nichtwäßriges Lösungsmittel für viele anorganische Salze (z.B. Chloride von Kupfer, Blei, Zink, Zinn, Kobalt, Eisen, Aluminium, Nickel; die Acetate der Alkalimetalle usw.). Es löst außerdem beispielsweise Gelatine, Glucose, Stärke, Polyvinylalkohol und Zelluloseacetate. Wegen seiner Bifunktionaltiät (Carbonyl- und Amid-Gruppe) ist Formamid zu zahlreichen Reaktionen der organischen Chemie befähigt. Es reagiert mit Formaldehyd zu einem Hydroxymethyl-Derivat, wird katalytisch zu Acrylnitril umgewandelt, zersetzt sich bei Temperaturen > 200°C zu Kohlenmonoxid, Ammoniak, Blausäure und Wasser und hydrolysiert sehr langsam bei Raumtemperatur - schneller bei höheren Temperaturen sowie in Gegenwart von Säuren oder Basen.

Formamid wird insbesondere als Lösungsmittel zur Herstellung von Vitaminen, Ameisensäure und Blausäure, zum Geschmeidigmachen von Leim und Papier, als Quellmittel in Dauerwellflüssigkeit usw., verwendet.

Die Herstellung von Formamid läuft in der Regel über die Grundstoffe Kohlenmonoxid und Ammoniak. Es handelt sich dabei um katalytische Umsetzungen, wobei als Katalysatoren insbesondere Metall-Katalysatoren, wie Ruthenium-Katalysatoren, oder Alkoholate wie Natriummethanolat oder Natriumethanolat in Frage kommen.

Bisher ausgeübte Verfahren, unter Verwendung von Alkoholaten als Katalysator, sind zweistufige Verfahren, bei denen in einer ersten Stufe Kohlenmonoxid und Methanol zu Methylformiat umgesetzt werden. Der Katalysator, meist Natriummethanolat, zersetzt sich unter Reaktionsbedingungen zu Natriumformiat. Dieses Salz lagert sich in der Regel in der verwendeten Apparatur ab, die dann regelmäßig zur Befreiung von Salz gespült werden muß - dies führt nachteilhafterweise zum Ausfall von Produktionszeit. In einer zweiten Stufe reagiert das Methylformiat unkatalysiert mit Ammoniak zu Formamid ab. Bei Herstellungsverfahren von Formamid handelt es sich meist um kontinuierliche Verfahren.

Im folgenden wird sich mit einem einstufigen Herstellungsverfahren befaßt. In der GB-A-2 216 035 werden die Reaktionsbedingungen so gewählt, daß das Lösungsmittel im Reaktor verbleibt. Es werden hochsiedende Lösungsmittel verwendet (mit einem höheren Siedepunkt als Formamid), so daß Formamid direkt aus dem Reaktionsgemisch abdestilliert werden kann und das Lösungsmittel nicht aufgearbeitet werden muß.
Außer der leichten Abtrennung des Produkts Formamid und der möglichst einfachen Regenerierung des Lösungsmittels besteht außerdem das Ziel, den Katalysator erneut verwenden zu können. Normalerweise wird der Katalysator, ein Alkoholat, d.h. ein homogener Katalysator, bei der Aufarbeitung aus dem Reaktor abgeführt und z.B. durch Quenchen zersetzt. Die DE-C-44 33 507 beschreibt die Synthese von Formamid unter Verwendung von Alkaliethylenglykolaten als Katalysator und von Ethylenglykolen als Lösungsmittel. Aufgrund des hohen Siedepunktes des Ethylenglykols, das gleichzeitig auch als Reaktand fungiert, kann Formamid leicht destillativ aus dem Reaktionsgemisch abdestilliert werden. Außerdem besteht die Möglichkeit, den Katalysator - das Alkaliethylenglykolat - zurückzugewinnen und anschließend wieder einzusetzen. Der hohe Siedepunkt des Lösungsmittels hat jedoch auch den Nachteil, daß dadurch die Zersetzung des Katalysators begünstigt wird.

Bei der Rückgewinnung dieser Alkoholate ist jedoch zu beachten, daß Verluste des Katalysators eintreten. Dies ist insbesondere darauf zurückzuführen, daß Alkoholate generell leicht mit Wasser hydrolysieren. Außerdem existieren andere Nebenreaktionen. Alkoholate können sich z.B. - begünstigt durch höhere Temperaturen - zersetzen. Ein wesentlicher Nachteil dieses Verfahrens besteht, wie bereits zuvor erwähnt, in der recht geringen Stabilität des verwendeten Katalysators.

DE 2710725 beschreibt ein Verfahren für die Formamidherstellung aber thematisiert hingegen weder die Rückgewinnung des Katalysators nach von nicht umgesetzten Edukten während der Aufarbeitung des Formamids.

Die Aufgabe der vorliegenden Erfindung ist, ein Verfahren hervorzubringen, bei dem die Direktsynthese von Formamid aus Kohlenmonoxid und Ammoniak so abläuft, daß das Produkt leicht aus der Reaktionsmischung abgetrennt werden kann und außerdem der Katalysator zurückgewonnen und wieder eingesetzt wird. Dabei soll ein Katalysator mit hoher katalytischer Aktivität und hoher Beständigkeit (insbesondere Temperatur- und chemische Beständigkeit) verwendet werden. Das Rezyklieren des Katalysators soll problemlos möglich sein. Während der Umsetzung und Aufarbeitung sollen nur geringe Verluste des Katalysators einhergehen. Außerdem sollen nicht umgesetzte Edukte, sowie Lösungsmittel zurückgewonnen werden können und für die Umsetzung wieder zur Verfugung stehen. Ein wesentlicher Aspekt ist, daß Kohlenmonoxid nicht nur als Reinstoff, sondern auch zusammen mit Wasserstoff oder anderen Inertgasen eingesetzt werden kann. Wasserstoff muß sich also unter den gewählten Verfahrensbedingungen inert verhalten.

Die Lösung dieser Aufgabe geht aus von dem Verfahren zur Herstellung von Formamid durch Umsetzung von Ammoniak und Kohlenmonoxid in Gegenwart mindestens eines Katalysators, und mindestens eines Alkohols in einem Reaktor gemäß Anspruch 1.

Unter Natriummethanolat und Natriumdiformylamid sollen nicht nur die Natriumsalze als solche verstanden werden, sondern alle vergleichbaren Salze, die Alkoholat bzw. Diformylamid bilden können - das Kation ist prinzipiell frei wählbar. Bevorzugte Alkoholate und Diformylamide sind entsprechende Alkali- und Erdalkalimetallsalze (zum Beispiel Kaliummethanolat oder Kaliumethanolat).

Die Umsetzung von Ammoniak und Kohlenmonoxid erfolgt nach folgendem vereinfachten Schema:

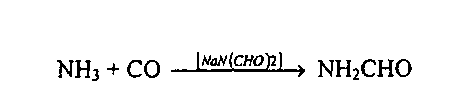

Bei den erfindungsgemäßen Herstellungsverfahren von Formamid erfolgt die Umsetzung meist bei Temperaturen von 50 bis 200°C, bevorzugt bei 100 bis 110°C. In der Regel findet dabei die Umsetzung bei Drücken zwischen 10 und 200 bar statt. Die vorstehend erwähnten Temperaturen und Drücke liegen in dem Reaktor vor, in dem die Umsetzung stattfindet.

Bei den erfindungsgemäßen Verfahren kann Kohlenmonoxid in Form eines Kohlenmonoxid und Wasserstoff enthaltenden Gemischs, bevorzugt Synthesegas, bereitgestellt werden. Das Kohlenmonoxid und Wasserstoff enthaltende Gemisch wird dann in der Regel dem Reaktor zugeführt. Es besteht also die Möglichkeit, wahlweise entweder reines Kohlenmonoxid oder ein kohlenmonoxidhaltiges Gas einzusetzen. Dabei kann auch Synthesegas mit einem hohen Wasserstoff anteil (> 50 %) eingesetzt werden. Dies wird dadurch ermöglicht, daß sich Wasserstoff unter den bevorzugten erfindungsgemäßen Reaktionsbedingungen (insbesondere bestimmt durch den Katalysator, durch die Temperatur, durch den Druck und durch das Lösungsmittel) inert verhält. Synthesegas ist in der Regel wesentlich preisgünstiger als reines Kohlenmonoxid, so daß durch dessen Verwendung ein Kostenvorteil entsteht.

Bei den erfindungsgemäßen Verfahren erfolgt die Umsetzung bevorzugt in Gegenwart mindestens eines Alkohols, bevorzugt in Gegenwart von Methanol.

Ein wesentlicher Schritt zum Hervorbringen des erfindungsgemäßen Verfahrens war möglicherweise, daß gefunden wurde, daß Natriummethanolat unter den vorstehend beschriebenen Bedingungen (Lösungsmittel, Temperatur, Druck) in Natriumdiformylamid umgewandelt wird - Natriumdiformylamid ist also ein Folgeprodukt von Natriummethanolat. Natriumdiformylamid ist ein wesentlich stabilerer Katalysator als Natriummethanolat. Es reagiert beispielsweise mit Wasser wesentlich langsamer als Natriummethanolat (ist also weniger Hydrolyseanfällig).

Ein weiterer wichtiger Vorteil des Natriumdiformylamids ist, daß es - im Gegensatz zu Alkoholaten - in dem Reaktionsgemisch unter den vorliegenden Reaktionsbedingungen keine unlöslichen Salze bildet, so daß die vorstehend aufgeführte Verstopfungsproblematik bei der Verwendung von Natriumdiformylamid nicht auftritt.

Die besondere Stabilität des Katalysators Natriumdiformylamid ermöglicht dessen Wiedergewinnung und den erneuten Einsatz bei der Umsetzung.

Das in dem Reaktor entstandene Reaktionsgemisch wird in der Regel direkt zur Aufarbeitung in die Apparatur geführt. In dieser Apparatur findet insbesondere Stofftrennung durch Destillation statt. Außerdem wird meist in der Apparatur ein großer Teil des in die Apparatur überführten Natriummethanolats (vorausgesetzt Natriummethanolat wird eingesetzt) zu Natriumdiformylamid umgesetzt. Dabei wird in der Regel zunächst der Austrag des Reaktors in einem Abscheider in flüssige und gasförmige Bestandteile aufgetrennt und der Flüssigaustrag in einer ersten Kolonne von Methanol befreit (sofern Methanol als Alkohol zum Einsatz kommt), welches in den Reaktor zurückgeführt wird. Meist wird dann nach Durchlaufen eines Wärmetauschers der Katalysator vom Produkt Formamid in einer zweiten Kolonne abgetrennt. In der Regel wird jedoch der Katalysator nicht als Reinstoff isoliert, sondern es wird eine Natriumdiformylamid enthaltende Mischung abgetrennt, die Formamid, bevorzugt mindestens 60 Gew.-% Formamid aufweist. Meist handelt es sich um eine ca. 20 gew.-%-ige Lösung des Katalysators in Formamid. Während in der Regel ein Teil dieser Lösung ausgeschleust wird, um die Anreicherung von Zersetzungsprodukten zu verhindern, wird bevorzugt der andere Teil dieser Lösung in den Reaktor zurückgeführt. Somit wird der Katalysator in diesem kontinuierlichen Herstellungsverfahren von Formamid wiedergewonnen und rezykliert.

Natriummethanolat ist das Vorprodukt des eigentlichen Katalysators Natriumdiformylamid - möglicherweise ist Natriumdiformylamid ein Reaktionsprodukt aus Natriummethanolat und Formamid. Die Summe der Konzentrationen von Natriummethanolat und Natriumdiformylamid im Reaktor beträgt meist 0,05 bis 1,0 mol/l, bevorzugt 0,2 bis 0,25 mol/l. Verluste von Natriummethanolat und/oder Natriumdiformylamid werden in der Regel durch Nachdosierung von Natriummethanolat in den Reaktor ausgeglichen. Bevorzugt wird Natriummethanolat in Form einer 30 gew.%-igen methanolischen Lösung zum Reaktionsgemisch gegeben. Prinzipiell ist eine Nachdosierung von Natriumdiformylamid auch möglich.

Nicht umgesetztes Kohlenmonoxid und/oder nicht umgesetzter Ammoniak und/oder Alkohol werden in der Regel aus der Apparatur abgeführt und in den Reaktor zurückgeführt.

Häufig wird als Alkohol Methanol eingesetzt. Dann liegt in der Regel ein molares Verhältnis von Ammoniak zu Kohlenmonoxid zu Methanol von 1,0:1,0 - 1,5:0,5 - 1,5 vor. Als besonders günstig erweist sich dabei das Verhältnis 1,0:1,25:1,0. Der in dem Reaktor vorliegende Druck liegt, wie vorstehend beschrieben, bevorzugt bei 10 bis 200 bar. Bei Verwendung von reinem Kohlenmonoxid ist dabei ein Druck von 40 bar und bei der Verwendung eines Kohlenmonoxidhaltigen Gases, wie Synthesegas, ein Druck von 120 bar bevorzugt.

Im folgenden soll die Erfindung anhand von Ausführungsbeispielen zusätzlich näher erläutert werden.

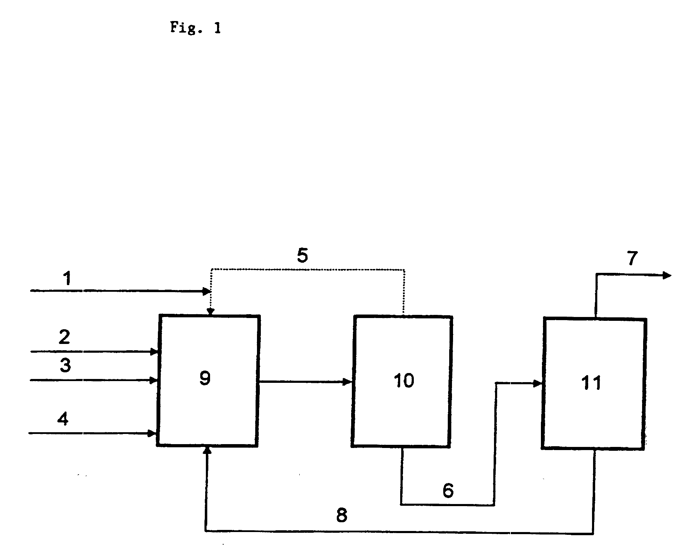

In der anliegenden Zeichnung zeigt Fig. 1 eine Skizze, die das Prinzip einer bevorzugten Ausführungsform einer Anlage darstellt, mit der das erfindungsgemäße Verfahren durchgeführt werden kann.

### Beispiel 1:

Der Reaktor 9 der Anlage besteht aus einem Rührautoklaven mit Scheibenrührer und Strömungsbrechern. Das Reaktorvolumen betrug 230 ml. Über die Zuleitung 2 wurde das Edukt Ammoniak flüssig in den Reaktor dosiert. Kohlenmonoxid wurde gasförmig über die Leitung 3 zudosiert. Methanol und die Katalysatorlösung (30 % m/m Natriummethanolat in Methanol) wurden über die Leitung 4 in den Reaktor gepumpt. Das Reaktionsgemisch wurde aus dem Reaktor in die Apparatur ausgeschleust, die im wesentlichen zwei Destillationsstufen 10, 11 enthält. Dabei wurde das Reaktionsgemisch zunächst auf ca. 5 bar entspannt, wobei der größte Teil des nicht umgesetzten Kohlenmonoxids entweicht. Die anschließende Aufarbeitung erfolgte zweistufig: In der ersten Stufe wurde Methanol bei Atmosphärendruck destillativ entfernt und anschließend über die Leitung 5 in den Reaktor zurückgeführt. In der zweiten Stufe 11 wurde unter reduziertem Druck (ungefähr 23 mbar) der Großteil des gebildeten Formamids über Kopf abdestilliert und über Leitung 7 abgeführt. Der Rest Formamid, aus dem Sumpf des zweiten Verdampfers, wurde mit dem darin gelösten Katalysator herausgefördert, und 10 % des Sumpfinhaltes wurden aus dem Reaktor ausgetragen. Die somit anfallenden Verluste wurden durch Zufuhr von frischem 30 gew.%-igem Natriummethanolat in Methanol ausgeglichen. Die Anlage wurde bei 40 bar, 100°C und einem Verhältnis der Edukte Ammoniak zu Kohlenmonoxid zu Methanol von 1,00:1,25:1,00 betrieben. Die durch-schnittliche Ausbeute an Formamid bezogen auf Ammoniak betrug 96 % und die Raum-Zeit-Ausbeute 400 g Formamid/l/h.

### Beispiel 2:

Analog zum Beispiel 1 wurde ein Versuch mit Synthesegas (Verhältnis Kohlenmonoxid zu Wasserstoff = 45:55) bei 120 bar und 100°C und einem Verhältnis der Edukte Ammoniak zu Kohlenmonoxid zu Methanol von 1,00:1,25:1,00 gefahren. Die durchschnittliche Ausbeute an Formamid betrug 96 % bezüglich Ammoniak mit einer Raum-Zeit-Ausbeute von 400 g Formamid/l/h.

### Beispiel 3:

Analog zum Beispiel 1 wurde ein Versuch mit Natriumdiformylamid in Methanol anstatt mit Natriummethylat durchgeführt. Die Reaktion wurde bei 40 bar und 100°C durchgeführt, wobei die Menge an Natriumdiformylamid 3 bis 4 Gew.-%, bezogen auf Formamid, betrug. Die durchschnittliche Ausbeute an Formamid betrug 92 % mit einer Raum-Zeit-Ausbeute von 370 g Formamid/l/h.

## Patentansprüche

1. Verfahren zur Herstellung von Formamid durch Umsetzung von Ammoniak und Kohlenmonoxid in Gegenwart mindestens eines Katalysators und mindestens eines Alkohols in einem Reaktor, **dadurch gekennzeichnet, daß** das Formamid enthaltende Reaktionsgemisch aus dem Reaktor in eine separate Apparatur überführt und dort aufgearbeitet wird und der als Natriummethanolat und/oder Natriumdiformylamid vorliegende Katalysator in dieser Apparatur abgetrennt und als Bestandteil einer Mischung, die Formamid enthält, in den Reaktor zurückgeführt wird.

2. Verfahren nach Anspruch 1, **dadurch gekennzeichnet, daß** der Katalysator als Bestandteil einer Mischung, die mindestens 60 Gew.-% Formamid enthält, aus der Apparatur in den Reaktor zurückgeführt wird.

3. Verfahren nach Anspruch 1 oder 2, **dadurch gekennzeichnet, dass** es sich bei der aus der Apparatur in den Reaktor zurückgeführten Mischung um eine ca. 20 gew.-%ige Lösung des Katalysators in Formamid handelt.

4. Verfahren nach einem der Ansprüche 1 bis 3, **dadurch gekennzeichnet, daß** der Alkohol Methanol ist.

5. Verfahren nach einem der Ansprüche 1 bis 4, **dadurch gekennzeichnet, daß** Kohlenmonoxid in Form eines Kohlenmonoxid und Wasserstoff enthaltenden Gemisches bereitgestellt wird.

6. Verfahren nach Anspruch 5, **dadurch gekennzeichnet, daß** das Kohlenmonoxid und Wasserstoff enthaltende Gemisch Synthesegas ist.

7. Verfahren nach einem der Ansprüche 1 bis 6, **dadurch gekennzeichnet, daß** nicht umgesetztes Kohlenmonoxid und/oder nicht umgesetzter Ammoniak und/oder Alkohol in der Apparatur abgetrennt und in den Reaktor rückgeführt werden.

8. Verfahren nach einem der Ansprüche 1 bis 7, **dadurch gekennzeichnet, daß** als Alkohol Methanol eingesetzt wird und im Reaktor ein molares Verhältnis von Ammoniak zu Kohlenmonoxid zu Methanol von 1,0:1,0 bis 1,5:0,5 bis 1,5 vorliegt.

9. Verfahren nach einem der Ansprüche 1 bis 8, **dadurch gekennzeichnet, daß** die Konzentration des Katalysators im Reaktor 0,05 bis 1,0 mol/l beträgt.

10. Verfahren nach einem der Ansprüche 1 bis 9, **dadurch gekennzeichnet, daß** in der Apparatur die Aufarbeitung durch Destillation erfolgt.

11. Verfahren nach einem der Ansprüche 1 bis 10, **dadurch gekennzeichnet, daß** in der Apparatur aus dem Reaktionsgemisch, enthaltend Formamid, Ammoniak, Kohlenmonoxid, Katalysator und mindestens einen Alkohol, in einer ersten Kolonne der (die) Alkohol(e) und in einer zweiten Kolonne der Katalysator abgetrennt und in den Reaktor zurückgeführt werden.

## Claims

1. A process for preparing formamide by reaction of ammonia and carbon monoxide in the presence of at least one catalyst, wherein the formamide-containing reaction mixture is transferred from the reactor to a separate apparatus and is worked up there and the catalyst present as sodium methoxide and/or sodium diformylamide is separated off in this apparatus and recirculated as constituent of a mixture comprising formamide to the reactor.

2. A process as claimed in claim 1, wherein the catalyst is recirculated from the apparatus to the reactor as constituent of a mixture comprising at least 60% by weight of formamide.

3. A process as claimed in claim 1 or 2, wherein the mixture recirculated from the apparatus to the reactor is an about 20% strength by weight solution of the catalyst in formamide.

4. A process as claimed in any of claims 1 to 3, wherein the alcohol is methanol.

5. A process as claimed in any of claims 1 to 4, wherein carbon monoxide is supplied in the form of a mixture comprising carbon monoxide and hydrogen.

6. A process as claimed in claim 5, wherein the mixture comprising carbon monoxide and hydrogen is synthesis gas.

7. A process as claimed in any of claims 1 to 6, wherein unreacted carbon monoxide and/or unreacted ammonia and/or alcohol are/is taken off from the apparatus and recirculated to the reactor.

8. A process as claimed in any of claims 1 to 7, wherein the alcohol used is methanol and the molar ratio of ammonia to carbon monoxide to methanol in the reactor is 1.0:1.0-1.5:0.5-1.5.

9. A process as claimed in any of claims 1 to 8, wherein the concentration of the catalyst in the reactor is from 0.05 to 1.0 mol/l.

10. A process as claimed in any of claims 1 to 9, wherein the work-up in the apparatus is carried out by distillation.

11. A process as claimed in any of claims 1 to 10, wherein, in the apparatus, the alcohol(s) is/are separated off from the reaction mixture comprising formamide, ammonia, carbon monoxide, catalyst and at least one alcohol in a first column and the catalyst is separated off in a second column and these are recirculated to the reactor.

## Revendications

1. Procédé de préparation de formamide par réaction de l'ammoniac et du monoxyde de carbone en présence d'au moins un catalyseur et au moins un alcool dans un réacteur, **caractérisé en ce que** le mélange réactionnel contenant du formamide est transféré du réacteur à un appareillage séparé et y est traité et que le catalyseur présent sous forme d'éthanolate de sodium et/ou de diformylamide de sodium est séparé dans cet appareillage et est renvoyé dans le réacteur à titre de composant d'un mélange contenant du formamide.

2. Procédé selon la revendication 1, **caractérisé en ce que** le catalyseur est renvoyé de l'appareillage dans le réacteur à titre de composant d'un mélange contenant au moins 60 % en poids de formamide.

3. Procédé selon la revendication 1 ou 2, **caractérisé en ce que** le mélange renvoyé de l'appareillage au réacteur est une solution à environ 20 % en poids du catalyseur dans du formamide.

4. Procédé selon l'une des revendications 1 à 3, **caractérisé en ce que** l'alcool est le méthanol.

5. Procédé selon l'une des revendications 1 à 4, **caractérisé en ce que** le monoxyde de carbone est mis à disposition sous la forme d'un mélange contenant du monoxyde de carbone et de l'hydrogène.

6. Procédé selon la revendication 5, **caractérisé en ce que** le mélange contenant le monoxyde de carbone et l'hydrogène est un gaz de synthèse.

7. Procédé selon l'une des revendications 1 à 6, **caractérisé en ce que** le monoxyde de carbone n'ayant pas réagi et/ou l'ammoniac n'ayant pas réagi et/ou l'alcool sont séparés dans l'appareillage et sont renvoyés dans le réacteur.

8. Procédé selon l'une des revendications 1 à 7, **caractérisé en ce que** l'on met en oeuvre à titre d'alcool du méthanol et qu'il existe un rapport molaire de l'ammoniac sur le monoxyde de carbone sur le méthanol de 1,0:1,0 à 1,5 :0,5 à 1,5 dans le réacteur.

9. Procédé selon l'une des revendications 1 à 8, **caractérisé en ce que** la concentration du catalyseur dans le réacteur va de 0,05 à 1,0 mol/l.

10. Procédé selon l'une des revendications 1 à 9, **caractérisé en ce que** le traitement se fait par distillation dans l'appareillage.

11. Procédé selon l'une des revendications 1 à 10, **caractérisé en ce que**, dans l'appareillage, l'alcool ou les alcools sont séparés dans une première colonne et le catalyseur dans une deuxième colonne, du mélange réactionnel contenant du formamide, de l'ammoniac, du monoxyde de carbone et au moins un alcool, et sont renvoyés dans le réacteur.
